(19) Europäisches Patentamt

European Patent Office

Office européen des brevets

(11) Veröffentlichungsnummer: **0 148 253 B1**

(12) **EUROPÄISCHE PATENTSCHRIFT**

(45) Veröffentlichungstag der Patentschrift: **02.12.92**

(21) Anmeldenummer: **84902709.9**

(22) Anmeldetag: **06.07.84**

(86) Internationale Anmeldenummer:
**PCT/EP84/00208**

(87) Internationale Veröffentlichungsnummer:
**WO 85/00291 (31.01.85 85/03)**

Verbunden mit 84107949.4/0131291
(europäische
Anmeldenummer/Veröffentlichungsnummer)
durch Entscheidung vom 18.09.87.

Die Akte enthält technische Angaben, die nach
dem Eingang der Anmeldung eingereicht wurden
und die nicht in dieser Patentschrift enthalten sind.

(51) Int. Cl.⁵: **A61L 25/00**, A61L 27/00,
C08K 7/18, //C08K3/32

(54) **IMPLANTATIONSMATERIALIEN.**

(30) Priorität: **12.07.83 DE 3325111**

(43) Veröffentlichungstag der Anmeldung:
**17.07.85 Patentblatt 85/29**

(45) Bekanntmachung des Hinweises auf die
Patenterteilung:
**02.12.92 Patentblatt 92/49**

(84) Benannte Vertragsstaaten:
**AT BE CH DE FR GB IT LI NL SE**

(56) Entgegenhaltungen:
**EP-A- 0 016 906**
**EP-A- 0 049 720**
**EP-A- 0 061 108**
**EP-A- 0 087 662**

(73) Patentinhaber: **MERCK PATENT GESELL-
SCHAFT MIT BESCHRÄNKTER HAFTUNG
Frankfurter Strasse 250 Postfach 4119
W-6100 Darmstadt(DE)**

(72) Erfinder: **DRAENERT, Klaus
Alte Landstrasse 26
W-8012 Ottobrunn(DE)**

## Beschreibung

Die Erfindung betrifft Implantationsmaterialien auf Basis von Polyacrylaten, die insbesondere als Knochenersatz-, Knochenverbund- und Prothesenverankerungsmaterialien Verwendung finden können.

Aus der EP-A1-16906 ist es bekannt, diesen auch als Knochenzemente bekannten Implantationsmaterialien resorbierbares Tricalciumphosphat beizumischen, um durch die am Rande des Implantats einsetzende Resorption Kanäle im Implantat zu schaffen, in die Knochengewebe einwachsen kann. Es kommt dadurch zu einer Verzahnung des Implantats mit dem umgebenden Knochen und damit zu einer verbesserten Langzeitstabilität. Durch die Verwendung relativ geringer Mengen an Tricalciumphosphat von 5 bis 35 Gewichtsprozent, insbesondere etwa 20 bis 25 Gewichtsprozent, kommt es im wesentlichen nur zur Ausbildung einer Randporosität, da die resorbierbaren Füllstoffteilchen im Implantat nicht so dicht gepackt sind, daß sie einander berühren und somit eine vollständige Resorption vom Rande her möglich wäre.

Es hat sich nun gezeigt, daß die im Innern des Implantats liegenden Füllstoffteilchen Ausgangspunkt für eine Zerstörung des Implantats sein können. Offenbar nehmen die Tricalciumphosphatteilchen an elastischen Bewegungen des Acrylatpolymeren, die bei Belastung und Entlastung der einzementierten Prothese stattfinden, nicht teil, so daß es an den eingelagerten Partikeln zu Streßsituationen kommt, die auf Dauer das Polymergefüge zerstören.

Eine Lösung dieses Problems in der Weise, daß der Füllstoffanteil so weit erhöht wird, daß die Teilchen einander berühren und somit vom Rand her vollständig resorbiert werden können, empfiehlt sich nicht, da dadurch die Kurszeitstabilität des Implantats selbst stark vermindert wird. Andererseits wird durch eine Randporosität bereits eine so gute Verankerung des Implantats erzielt, daß auf eine vollständige Durchwachsung des Implantats mit Knochengewebe mit dem Ziel einer guten Langzeitstabilität verzichtet werden kann.

Es bestand daher die Aufgabe, ein Füllmaterial zu finden, das auf der Basis von Tricalciumphosphat aufgebaut ist und in den aus der EP-A1 16906 bekannten Mengen- und Teilchengrößen dem Zement beigemischt werden kann, das aber Streßsituationen zwischen Füllstoffpartikel und Polymerverbund weitgehend vermeidet.

Diese Aufgabe wurde durch die vorliegende Erfindung gelöst.

Gegenstand der Erfindung sind daher Implantationsmaterialien auf Basis von Polyacrylaten, die 5 bis 35 Gewichtsprozent von Teilchen auf Basis von resorbierbarem Tricalciumsphosphat mit einer Teilchengröße zwischen 20 und 300 $\mu$m enthalten, die dadurch gekennzeichnet sind, daß diese Teilchen überwiegend annähernd Kugelgestalt aufweisen.

Gegenstand der Erfindung sind auch resorbierbare Füllstoffpartikel auf Basis von Tricalciumphosphat zur Verwendung in Knochenzementen, die dadurch gekennzeichnet sind, daß sie eine Teilchengröße zwischen 50 und 300 $\mu$m besitzen und überwiegend annähernd Kugelgestalt aufweisen.

Die Erfindung bringt eine Reihe von Vorteilen mit sich. Ganz wesentlich ist die Verminderung der Streßbelastung, die von einem unregelmäßigen Teilchen mit Ecken und Kanten bei elastischen Bewegungen des Zements ausgehen. Bei einem weitgehend runden Teilchen kann dagegen in gewissem Maße eine Verschiebung der Grenzflächen zwischen Füllstoffteilchen und Acrylatpolymer stattfinden, ohne daß es dabei zu Belastungen des Polymeren kommt. Auch bei einer reinen Druckbelastung des Zements ist die Kugelform von wesentlichem Vorteil, da hier sowohl die Druckaufnahme als auch die Weitergabe gleichmäßig verteilt über eine große Fläche erfolgt und nicht auf wenige exponierte Punkte beschränkt ist.

Die Herstellung der erfindungsgemäßen Füllstoffe kann auf verschiedene Weise erfolgen. Als Ausgangsmaterial wird ein hochporöses Tricalciumphosphat mit einer Porosität von etwa 50 bis 80 % bevorzugt. Es hat sich gezeigt, daß ein hochporöses Tricalciumphosphat weitaus schneller resorbiert wird als ein durch Sinterung in seinem Porenvolumen reduziertes Tricalciumphosphat. Um zu verhindern, daß beim Vermischen der Komponenten des Knochenzements flüssiges Monomeres in die Poren des Tricalciumphosphats aufgenommen wird und dort auspolymerisiert werden die Poren mit einem körperverträglichen resorbierbaren Material gefüllt, das mit dem Acrlyatmonomeren nicht mischbar ist. Diese Porenfüllung kann durch schnell resorbierbare Substanzen, wie z. B. Glycerin, geschehen, es ist aber auch möglich, dazu resorbierbare Polymere, wie z. B. Polylactide, Polyglycolide, Polyhydrocycarbonsäuren oder Polyaminosäuren zu verwenden. Eine Porenfüllung mit resorbierbaren Polymeren hat den Vorteil, daß die Teilchen eine höhere mechanische Stabilität besitzen, was insbesondere in bezug auf die im Innern des Knochenzements verbleibenden Teilchen wesentlich ist.

Eine weitere bevorzugte Möglichkeit zur Herstellung der erfindungsgemäßen Füllstoffe besteht darin, von einem feinteiligen pulverförmigen Tricalciumphosphat mit einer Teilchengröße etwa im Bereich von 1 bis 5 $\mu$m auszugehen und dieses mit einem körperverträglichen resorbierbaren Material zu weitgehend kugelförmigen Teilchen der Größe 20 bis 300 $\mu$m, insbesondere etwa 150 bis 250 $\mu$m zu verkleben. Als Matrixmaterial für das pulverförmige Tricalciumphosphat kommen beispielsweise resorbierbare Polymere, wie Polylactid, Polyglycolid, Polyhydroxycarbonsäuren, Polyaminosäuren und Polyester in Frage.

Diese auf Basis von pulverförmigem Tricalciumphosphat aufgebauten Füllstoffpartikel sind besonders vorteilhaft, da dieses Verbundmaterial aus einer Polymermatrix und Tricalciumphosphatpulver eine weitgehend ähnliche Elastizität wie das den Knochenzement bildende Acrylatpolymere besitzt. Bei elastischer Bewegung des Knochenzements im Körper können daher die weitgehend isoelastischen Füllstoffpartikel sich in gleicher Weise verformen, so daß es zu keinerlei Oberflächenverschiebung zwischen Füllstoffpartikel und Knochenzement kommen kann. Auf diese Weise ist eine optimale mechanische Verträglichkeit des Füllstoffs mit dem Zement gegeben.

Von den rein mechanischen Eigenschaften des Zements her wäre es denkbar, die Füllstoffe vollständig aus einem resorbierbaren Polymeren herzustellen. In Bezug auf die biologischen Eigenschaften des Implantats jedoch ist die Anwesenheit von Tricalciumphosphat sehr erwünscht. Die erfindungsgemäßen Füllstoffpartikel enthalten daher in der Regel etwa 20 bis 90 % Tricalciumphosphat, insbesondere etwa 30 bis 50 %.

Der Begriff Tricalciumphosphat, der in der vorliegenden Anmeldung gebraucht wird, ist als Oberbegriff zu einer Anzahl von verschiedenen, im wesentlichen durch die chemische Formel $Ca_3(PO_4)_2$ zu beschreibende Materialien zu verstehen, wobei das Verhältnis Calcium zu Phosphor annähernd 3 : 2 beträgt.

Neben den reinen Tricalciumphosphaten, wie z. B. $\alpha$- oder $\beta$-Whitlockit sollen jedoch auch die nur annähernd durch die Formel $Ca_3(PO_4)_2$ zu beschreibenden Materialien, wie z. B. Apatite oder Phosphorit umfaßt sein. Insbesondere ist Hydroxylapatit ein bevorzugtes Material. Auf jeden Fall soll das Tricalciumphosphat im Körper resorbierbar sein.

Die erfindungsgemäßen Füllstoffe werden analog den in der EP-A1 16906 beschriebenen Verfahren in Knochenzemente eingearbeitet und für die dort beschriebenen Zwecke verwendet. Durch die geschilderten Vorteile haben Implantationsmaterialien, die die erfindungsgemäßen Füllstoffe enthalten, eine wesentlich verbesserte Langzeitstabilität, so daß mit der vorliegenden Erfindung ein wesentlicher Forschritt auf dem Gebiet der Implantationsmaterialien erzielt wird.

**Patentansprüche**

1. Implantationsmaterialien auf Basis von Polyacrylaten, die 5 bis 35 Gewichtsprozent von Teilchen auf Basis von resorbierbarem Tricalciumphosphat mit einer Teilchengröße zwischen 20 und 300 $\mu$m enthalten, dadurch gekennzeichnet, daß diese Teilchen überwiegend annähernd Kugelgestalt aufweisen.

2. Implantationsmaterialien nach Anspruch 1, dadurch gekennzeichnet, daß die Teilchen aus einem hochporösen Tricalciumphosphat bestehen, dessen Poren mit einem resorbierbaren körperverträglichen Stoff gefüllt sind.

3. Implantationsmaterialien nach Anspruch 1, dadurch gekennzeichnet, daß die Teilchen aus Tricalciumphosphatpulver bestehen, das mit einem resorbierbaren körperverträglichen Stoff verklebt ist.

4. Implantationsmaterialien nach Anspruch 2 oder 3, dadurch gekennzeichnet, daß als resorbierbarer körperverträglicher Stoff eine Polyaminosäure, oder Glycerin verwendet wird.

5. Implantationsmaterialien nach Anspruch 2, dadurch gekennzeichnet, daß ein Tricalciumphosphat mit einer Porosität von 50 bis 80 % verwendet wird.

6. Resorbierbare Füllstoffpartikel auf Basis von Tricalciumphosphat zur Verwendung in Knochenzementen, dadurch gekennzeichnet, daß sie eine Teilchengröße zwischen 20 und 300 $\mu$m besitzen und überwiegend annähernd Kugelgestalt aufweisen.

7. Füllstoffpartikel nach Anspruch 6, dadurch gekennzeichnet, daß die Teilchen ein Tricalciumphosphat mit einer Porosität von 50 bis 80 % enthalten.

8. Füllstoffpartikel nach Anspruch 6 oder 7, dadurch gekennzeichnet, daß neben Tricalciumphosphat ein körperverträglicher resorbierbarer Stoff enthalten ist.

9. Verwendung eines Füllstoffs nach Anspruch 6 zur Herstellung von Knochenzementen.

**Claims**

1. Materials for implantation based on polyacrylates, which contain 5 to 35 per cent by weight of particles based on absorbable tricalcium phosphate having a particle size between 20 and 300 $\mu$m, characterised in that these particles predominantly have an approximately spherical shape.

2. Materials for implantation according to Claim 1, characterised in that the particles are composed of a highly porous tricalcium phosphate whose pores are filled with an absorbable substance which is tolerated by the body.

3. Materials for implantation according to Claim 1, characterised in that the particles consist of tricalcium phosphate powder which has been bonded with an absorbable substance which is tolerated by the body.

4. Materials for implantation according to Claim 2 or 2, characterised in that a polyaminoacid or glycerol is used as the absorbable substance which is tolerated by the body.

5. Materials for implantation according to Claim 2, characterized in that the tricalcium phosphate used has a porosity of 50 to 80%.

6. Particles of absorbable filler based on tricalcium phosphate, for use in bone cements, characterised in that they have a particle size between 20 and 300 $\mu$m, and predominantly have an approximately spherical shape.

7. Particles of filler according to Claim 6, characterised in that the particles contain a tricalcium phosphate having a porosity of 50 to 80%.

8. Particles of filler according to Claim 6 or 7, characterised in that, in addition to tricalcium phosphate, they contain an absorbable substance which is tolerated by the body.

9. Use of a filler according to Claim 6 for the preparation of bone cements.

**Revendications**

1. Matériaux d'implantation à base de polyacrylates, contenant 5 à 35% en poids de particules à base de phosphate tricalcique résorbable, à une dimension de particule de 20 à 300 $\mu$m, caractérisés en ce que ces particules ont principalement une forme à peu près sphérique.

2. Matériaux d'implantation selon revendication 1, caractérisés en ce que les particules consistent en un phosphate tricalcique à forte porosité dont les pores ont été remplis d'une substance résorbable tolérée par l'organisme.

3. Matériaux d'implantation selon la revendication 1, caractérisés en ce que les particules consistent en poudre de phosphate tricalcique qu'on a agglomérée à l'aide d'une substance résorbable tolérée par l'organisme.

4. Matériaux d'implantation selon revendication 2 ou 3, caractérisés en ce que l'on a utilisé en tant que substance résorbable tolérée par l'organisme un polyaminoacide ou le glycérol.

5. Matériaux d'implantation selon revendication 2, caractérisés en ce que l'on utilise un phosphate tricalcique à une porosité de 50 à 80%.

6. Particules de matière de charge résorbable à base de phosphate tricalcique pour l'utilisation dans des ciments pour os, caractérisées en ce qu'elles ont une dimension de particule de 20 à 300 $\mu$m et principalement une forme à peu près sphérique.

7. Particules de matière de charge selon revendication 6, caractérisées en ce que les particules contiennent un phosphate tricalcique ayant une porosité de 50 à 80%.

8. Particules de matière de charge selon revendication 6 ou 7, caractérisées en ce qu'elles contiennent, en plus du phosphate tricalcique, une substance résorbable tolérée par l'organisme.

9. Utilisation d'une matière de charge selon revendication 6 pour la préparation de ciments pour os.